# EUROPEAN PATENT APPLICATION

(11) **EP 2 602 624 A1**
(43) Date of publication of application: **12.06.2013**
(21) Application number: 13157838.7
(22) Date of filing: 03.08.2007
(51) Int. Cl.: G01N 33/68

(54) **Diagnostic test to exclude significant renal injury**

(30) Priority: 07.08.2006 US 836088 P
(62) Divisional of application: 07785730.8
(71) Applicant: Antibodyshop A/S, 2820 Gentofte (DK)
(72) Inventor: Uttenthal, Lars Otto, 37002 Salamanca (ES); Bangert, Kristian, 2840 Holte (DK)
(74) Representative: Høiberg A/S

(57) **Abstract**

Methods for determining the risk of developing acute renal failure in a human subject by measuring human neutrophil gelatinase-associated lipocalin (NGAL) are provided.

## Description

### Field of the invention

The present invention relates to the identification of those individuals within a certain population or group of patients who, while being at general risk of suffering renal injury, do not have significant renal injury and are not at immediate risk of developing acute renal failure. As such it is relevant to internal medicine and in particular to critical or intensive care medicine, but also to surgery, oncology and diagnostic imaging, where procedures that may injure the kidneys are carried out.

### Background of the invention

Acute renal failure (ARF) is a sudden loss of functionality of kidney tissue that may sometimes progress to chronic renal failure. If managed appropriately the kidney may regain full functionality. Early intervention maximizes the chances of renal recovery (Molitoris, 2003). To permit such early intervention, it is necessary either to make an early diagnosis or to treat on suspicion.

At present the diagnosis of ARF is based on clinical signs such as oliguria and fluid retention, combined with the results of chemical tests on blood and urine showing a low sodium excretion, raised and rising plasma levels of creatinine and a decline in creatinine clearance. These tests are essentially functional tests that demonstrate the consequences of a significant degree of renal failure and therefore indicate the presence of the established condition. With a view to detecting an earlier stage of the pathophysiology of ARF, various markers of renal tubular damage have been analyzed in urine. Of these, alpha 1-microglobulin, beta 2-microglobulin (Penders and Delanghe, 2004) and N-acetyl-beta-D-glucosaminidase (NAG) (Kotanko et al., 2000) are elevated in cases of established acute tubular necrosis (ATN), but may first appear as late as 4 or 5 days after the ischemic or toxic insult in experimental animals. Kidney injury molecule-1 (KIM-1) is also elevated in the urine of patients with ATN, but the time course, though probably early, has not been established in human patients (Han et al., 2002). Cysteine rich protein 61 (CYR61) peaks in the urine at 6 to 9 hours after ischemic injury in experimental animals (Muramatsu et al., 2002). However, levels in humans are still unknown. Other markers that have been studied include clusterin (Aulitzky et al., 1992) and lipocalin-type prostaglandin D synthase (L-PGDS) (Tsuchida et al., 2004), but their usefulness for the early diagnosis or prediction of ATN is still unclear. As a result of the generally slow time course of appearance of these marker molecules in the urine after renal ischemia or exposure of the kidney to nephrotoxic agents, they have not come into general use to diagnose early or impending renal disorders resulting from these insults. As a result, the patient with ARF is not treated until the condition has progressed to a relatively late stage when a reliable diagnosis is obtainable by the current functional tests.

Treating a condition on suspicion, i.e. before a diagnosis is established, leads to overtreatment, i.e. treatment applied to a condition that is not in fact present. Overtreatment increases costs and may put individuals at risk of unnecessary side effects. On the other hand, treatment on suspicion may lead to earlier treatment of the condition if it is present and include more individuals that will benefit from the treatment. Thus the overall value of a policy of treatment on suspicion depends on the proportion of true cases among the suspected individuals, and the disadvantages increase with an increasing proportion of false positive suspected diagnoses. In the case of ARF there are as yet no accepted treatments specifically directed at restituting the renal pathology. Current treatment consists essentially of optimizing the prerenal conditions necessary for good renal function, such as maintaining and improving the blood supply to the kidneys, eliminating any postrenal factors such as obstruction of urinary flow, and avoiding aggravating factors such as the use of nephrotoxic drugs. This means that the concept of overtreatment of ARF hardly applies until specific treatments of renal pathology are introduced. However, the establishment of reliable *in vitro* diagnostic tests of renal pathology will be a necessary adjunct to the development, monitoring and control of such specific treatments.

The present invention relates to the diagnostic use of a new marker molecule for renal pathology that appears in both blood and urine, to exclude the presence of significant renal tubular injury in a population of patients at risk of such injury. The marker molecule is neutrophil gelatinase-associated lipocalin (NGAL), also known as neutrophil lipocalin (NL; HNL in the case of human neutrophil lipocalin), lipocalin 2 (LCN2), 25-kDa alpha 2-microglobulin-related protein (in the rat) or 24P3 (in the mouse). In the rat, it has also been referred to as neu-related lipocalin (NRL), as its gene is overexpressed in mammary tumors initiated by the neu (HER2/c-erbB-2) oncogene (Stoesz and Gould, 1995). NGAL is a 25-kDa glycoprotein first isolated from the granules of neutrophil polymorphonuclear leukocytes (Triebel et al., 1992; Kjeldsen et al., 1993). It contains a disulfide bridge and forms a proportion of dimers and a smaller proportion of trimers. It is associated with 92-kDa human neutrophil type IV collagenase, also called matrix metalloproteinase 9 (MMP-9) or gelatinase B, either as a monomer forming a complex of apparent kDa 115, (Monier et al., 2000; Yan et al., 2001) or as a dimer, forming a complex of apparent kDa 125 (Yan et al., 2001). These complexes have been identified in the urine of patients with a variety of cancers, including cancers of the prostate, bladder, kidney and breast.

NGAL was initially disclosed as a marker of neutrophil activation, being released into the blood when invading microorganisms, in particular pyogenic bacteria, cause degranulation of the neutrophils and exocytosis of the granule proteins. As such, the measurement of elevated levels of NGAL in a plasma or serum sample from a human is believed to be indicative of the individual suffering from an inflammation, especially one caused by a bacterial infection (Venge, 2000; U.S. Patent 6,136,526; PCT application WO95/29404). In this respect, but in contradiction to its claimed specific derivation from neutrophils, NGAL (24P3) was identified as an acute phase protein of type 1 in the mouse, where expression was mainly located in the liver during the acute phase response (Liu and Nilsen-Hamilton, 1995).

US patent application 2004/0219603 describes the use of NGAL as a urinary biomarker for detecting the early onset of renal tubular cell injury. However, it is not described whether or how renal tubular cell injury can be discriminated from systemic inflammation, or bacterial infection, or cancers as the cause of the elevated NGAL level.

### Summary of the invention

It has now surprisingly been found that, in critically ill patients at risk of renal injury, normal or only moderately increased NGAL levels in bodily fluids such as urine and plasma are found exclusively in patients without ARF, whereas higher NGAL levels may be found both in patients with ARF or destined to develop ARF and in a few patients without ARF and who are not destined to develop ARF. Thus a high level of NGAL is not certainly diagnostic of ARF but can identify patients who are categorized as being "at high risk of developing or having ARF", whereas a normal or only moderately elevated level of NGAL identifies patients who are categorized as "not at risk of developing or having ARF".

Normally, ARF is treated after a positive diagnosis, but as this diagnosis is only obtainable after some time, typically one or more days after the pathological insult that precipitated ARF, and as early diagnosis of ARF is important for successful management of this condition, the present invention addresses this problem by employing the opposite strategy: identifying patients at risk of having or developing ARF by means of a diagnosis of exclusion that identifies the patients who are not at risk of having or developing ARF. Patients in the group at risk of developing ARF can accordingly be subjected to a closer diagnostic surveillance and/or earlier intervention than would otherwise be the case.

The identification of patients who do not have ARF and are not at immediate risk of developing ARF is achieved by determining the concentration of NGAL in a sample of bodily fluid from the individual and comparing the result to a cutoff value representing the upper limit of NGAL concentrations reached in critically ill patients who have not developed ARF at any time during the course of their illness. An NGAL concentration below this value indicates that the patient is not at immediate risk of developing ARF. An NGAL concentration above this value indicates that the patient is at risk of having or developing ARF, the risk rising with magnitude of the NGAL elevation.

The methods of the present invention are particularly useful in patients admitted to intensive or critical care departments and will also be useful in patients who not being critically ill have suffered a well-defined insult such as a surgical operation that may lead to ischemic injury of the kidney or exposure to nephrotoxic agents such as the intravenous administration of contrast media for diagnostic imaging or the administration of nephrotoxic chemotherapeutic agents. In these cases the identification of those patients whose kidneys have not been significantly affected by the procedure and who are therefore not at risk of ARF will allow the attending physicians to focus on an earlier and more intensive intervention in those other patients who are at risk of developing ARF.

Levels of NGAL in bodily fluids are preferably determined by an immunochemical method. Examples of such methods include, but are in no way limited to a sandwich ELISA (enzyme-linked immunosorbent assay), a lateral flow immunochromatographic method, a dipstick, or an automated immunochemical method based on antibody-coated microparticles.

### Description of the drawings

### Figure 1

Receiver operating characteristics (ROC) curve for maximal urinary NGAL values with respect to the diagnosis of acute renal failure (ARF) in 125 adult patients admitted to a hospital intensive care unit.

### Detailed Description of the Invention

In a study of unselected adult patients admitted to intensive care, it has been found that concentrations of NGAL in samples of urine and plasma are in most cases only moderately elevated by infective, inflammatory or cancerous states, if these are not complicated by ARF. However, NGAL levels are markedly elevated in patients with ARF or destined to develop ARF within the next 24 hours. The receiver operating characteristics (ROC) curve for the maximal urinary concentration values with respect to the diagnosis of ARF was asymmetrical with respect to the asymptotes corresponding to 100% diagnostic sensitivity and 100% diagnostic specificity for ARF, showing that NGAL determinations could provide greater diagnostic sensitivity with less loss of diagnostic specificity than *vice versa.* A cutoff value for the NGAL concentration could be chosen that gave 100% sensitivity for the diagnosis of ARF, while retaining a diagnostic specificity of 74%. On the other hand, the lowest cutoff value that gave 100% diagnostic specificity for ARF gave a diagnostic sensitivity of 56%. The cutoff value giving 100% diagnostic sensitivity corresponds to a value between the highest value found in the patients who did not develop ARF and the lowest value found in the patients who did develop ARF. Using this value or indeed any lower value as the cutoff gives a diagnostic test with a negative predictive value of 100%, i.e. predicting that patients from this population with a urinary NGAL concentration below this value do not have ARF.

Similar results were obtained with maximal plasma concentrations of NGAL, making it possible also to select a cutoff value for plasma concentrations that gave a negative predictive value of 100%.

Accordingly, the present invention relates to measurement of NGAL in a sample of bodily fluid, preferably urine or plasma, in order to determine the likelihood of ARF in a human subject, whereby it is possible to discriminate between a subject who is "not at risk of having or developing ARF" and a subject who is "at risk of having or developing ARF", the said invention comprising the steps of
i) determining the concentration of human NGAL in a sample of bodily fluid from the subject
ii) comparing said concentration with a predetermined cutoff value chosen as the highest value found in subjects that have not developed ARF during the course of their illness, so that an NGAL concentration below the cutoff value categorizes the subject as not having or not being at immediate risk of developing ARF.

It will be evident to those skilled in the art that the cutoff level so determined will depend on the characteristics of the patient population or group to which the test is applied. It is possible that certain patient groups that are not critically ill, such as patients subjected to elective surgery, cancer chemotherapy or diagnostic imaging with intravenous contrast agents, may require a lower cutoff value to provide a negative predictive value of 100%.

The cutoff level below which the urinary concentration of NGAL has a negative predictive value of 100% for ARF in a determined group of patients is preferably a concentration of 250 ng/mL or less, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL, or any value between 100 ng/mL and 50 ng/mL, such as 75 ng/mL.

In another embodiment, the present invention comprises the steps of measuring the concentration of NGAL in a sample of plasma or serum from the subject to be diagnosed, and comparing the measured concentration with a selected cutoff value below which the plasma or serum concentration has a negative predictive value of 100% for ARF in the group of patients to which the subject belongs. If the measured NGAL concentration is below the cutoff level, this is an indication that the subject has not suffered renal injury and is not at immediate risk of developing ARF.

The cutoff level below which the NGAL concentration in plasma or serum has a negative predictive value of 100% for ARF in a determined group of patients is similar to that for urine and is preferably a concentration of 250 ng/mL or less, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL.

A further aspect of the present invention is that the method can be used to monitor patients throughout the course of an illness or at various times after a diagnostic or therapeutic intervention that carries a risk of provoking ARF. Comparison of the measured NGAL levels with the cutoff value will determine when the patient leaves the category of "not at risk of having or developing ARF" and enters the category of "at risk of having or developing ARF". The intervals at which samples of bodily fluids are taken for monitoring can be short, thus providing the earliest possible indication of the change of risk category and thus permitting the early institution of more intensive surveillance and any therapeutic measures. Monitoring of NGAL levels in bodily fluids for this purpose is preferably carried out at intervals not longer than 24 hours, and more preferably at shorter intervals down to a suggested period of not longer than 3 h, or even shorter, such as 30 minutes or 1 hour, for instance if a potential renal insult is known to have occurred, e.g. during a surgical or medical procedure.

Measurement of human NGAL in a sample of bodily fluid, such as a sample of urine or plasma, can be performed by any method that provides satisfactory analytical specificity, sensitivity and precision. Preferred methods are binding assays using one or more binding molecules specific to human NGAL. Such binding molecules include, but are not limited to, polyclonal or monoclonal antibodies against NGAL or specific NGAL binding molecules generated by other means.

In a preferred method, monoclonal antibodies raised against recombinant human NGAL are used. One antibody is linked to a solid support to capture NGAL from a sample, such as a urine sample, while the other is linked to a label such as a dye complex, or biotin or enzyme that can be detected by any of many methods known to those skilled in the art. The solid support may e.g. be a polystyrene or polyvinyl chloride surface for enzyme-linked immunosorbent assay (ELISA), or latex (polystyrene) particles, or a filter frit composed of compressed polyethylene particles, or a porous nitrocellulose matrix, or indeed any suitable support used in immunochemical analyses.

A preferred means for measuring NGAL in accordance with the present invention in a sample of human urine includes a dipstick, lateral flow (immunochromatographic) or minicolumn test, which allows for the rapid, near-patient analysis of a sample. As will be understood by those of skill in the art upon reading this disclosure, however, other means for measuring NGAL can be used, including automated methods in central laboratories in which the apparatus permits the random access of samples for urgent analysis.

In a preferred embodiment, the method of the invention does not comprise a surgical, therapeutic or diagnostic step practiced on the human body.

The following non-limiting examples are provided to further illustrate the present invention.

### EXAMPLES

### Example 1: NGAL Dipstick Test

The analytical area of a dipstick comprised of a polystyrene surface is coated with a capture antibody against human NGAL. An aliquot of the centrifuged, diluted sample is added to a solution of enzyme-labeled detection antibody against NGAL in the first tube, into which the dipstick is immersed. Complexes of enzyme-labeled detection antibody with NGAL are bound to the dipstick, which is then washed with tap water and placed in a chromogenic substrate solution in a second tube. The color developed in the substrate solution within a given time is read either by eye and compared with a chart of color intensities which indicates the concentration of NGAL in the urine sample, or in a simple colorimeter that can, for example, be programmed to indicate the NGAL concentration directly.

### Example 2: NGAL Lateral Flow Device

A lateral flow device comprised of a strip of porous nitrocellulose is coated near its distal end with a capture antibody against NGAL applied as a transverse band. A further transverse band of antibody against antibodies of the species from which the detection antibody is derived is placed distally to the capture antibody band and serves as a control of strip function. The proximal end of the strip contains the detection antibody against NGAL adsorbed or linked to labeled polystyrene particles or particles of dye complex. When an aliquot of the centrifuged urine sample is applied to the proximal end of the strip, the labeled particles attached to detection antibody travel along the strip by capillary attraction. When reaching the band of capture antibody, only those particles which have bound NGAL will be retained, giving rise to a detectable band. Particles reaching the control band of antibody against the detection antibody will produce a detectable band whether or not any NGAL has been bound. The intensity of the labeled bands can be read by eye in the case of colored particles or by means of the appropriate detection device for the label used. A positive result is indicated by color development or the accumulation of label in both bands, while a negative result is indicated by color development or other label only in the control band. Failure of color development or other label in the control band indicates inadequate strip function. The sensitivity of the test can be regulated by the dilution of the sample applied, which is adjusted so that only NGAL concentrations above the determined cutoff values give rise to a positive result. The sensitivity of the test can also be adjusted by linking the detection antibody to a mixture of labeled and unlabeled particles. Batches of strips can be pre-calibrated and equipped with a calibration code that can be read by the detection device, so that a quantitative or semi-quantitative result can be read from the device. Many variations of the individual aspects of this lateral flow technology are possible, as known to those skilled in the art.

### Example 3: NGAL Minicolumn Test

A minicolumn contains a frit made of compressed polyethylene particles allowing the passage of fluid and cells. The frit is coated with capture antibody against human NGAL. The minicolumn is incorporated into a device, which by means of automated liquid handling allows the diluted sample to be applied at a fixed flow rate and volume, followed by detection antibody complexed with dye. After the passage of wash solution, the color intensity of the frit is read by light diffusion photometry. The batches of frits are pre-calibrated and the minicolumns equipped with a calibration code that can be read by the device, so that a quantitative result can be displayed by the instrument without the need for prior calibration with standards.

### Example 4: NGAL Sandwich ELISA

Purified recombinant human NGAL for use as a standard and as calibrator material was prepared as described by Kjeldsen et al. (1996). Antibodies against NGAL were those described by Kjeldsen et al. (1993; 1996). Polystyrene ELISA plates were coated overnight at 4°C with antibody 211-1 at a concentration of 2 µg/mL in 0.05 M sodium carbonate buffer, pH 9.4, applied at 100 µL/well. The wells were emptied, washed 3 times with wash buffer of phosphate-buffered saline, pH 7.4, containing 0.05% Tween 20, and blotted. Dilutions of calibrator and samples in dilution buffer (wash buffer containing bovine albumin at 0.1 mg/mL) were applied to the wells in 100-µL volumes and incubated for 1 hour at room temperature on a shaking table. The wells were then emptied, washed and blotted as before. Biotinylated antibody 211-2 at 0.25 µg/mL in dilution buffer was added to each well at 100 µL/well and incubated for 1 hour at room temperature on a shaking table. The wells were then emptied, washed and blotted as before. A complex of horseradish peroxidase and streptavidin (Zymed, CA) at a dilution of 1/2000 in dilution buffer was added to each well at 100 µL/well and incubated for 1 hour at room temperature on a shaking table. The wells were then emptied, washed and blotted as before. A substrate solution containing tetramethylbenzidine and peroxide (TMB-ONE, Kem-En-Tech, Denmark) was then applied to each well at 100 µL/well and incubated at room temperature in the dark for exactly 8 minutes, after which the color reaction was stopped by adding 50 µL of 1 M sulfuric acid to each well. The light absorbances of the wells at a wavelength of 450 nm were then read in an ELISA plate reader, subtracting the light absorbances at 650 nm. The concentrations of NGAL in the samples were then calculated from the standard curve generated from the light absorbance readings of the calibrators of known concentration.

The assay had a range of 0.02 ng/mL to 1 ng/mL, with a detection limit (95% confidence limit of difference from zero) of 2.4 pg/mL, and showed parallelism between dilutions of purified calibrator and samples. The concentration of NGAL was 90 ng/mL in a pool of normal human serum and 5.4 ng/mL in a pool of normal human urine.

### References

Aulitzky WK, Schlegel PN, Wu DF, Cheng CY, Chen CL, Li PS, Goldstein M, Reidenberg M, Bardin CW (1992) Measurement of urinary clusterin as an index of nephrotoxicity. Proc Soc Exp Biol Med 199:93-96.
Han WK, Bailly V, Abichandani R, Thadhani R, Bonventre JV (2002) Kidney Injury Molecule-1 (KIM-1): a novel biomarker for human renal proximal tubule injury. Kidney Int 62:237-244.
Kjeldsen L, Johnsen AH, Sengelov H, Borregaard N (1993) Isolation and primary structure of NGAL, a novel protein associated with human neutrophil gelatinase. J Biol Chem 268:10425-10432.
Kjeldsen L, Koch C, Arnljots K, Borregaard N (1996) Characterization of two ELISAs for NGAL, a newly described lipocalin in human neutrophils. J Immunol Methods 198:155-164.
Kotanko P, Margreiter R, Pfaller W (2000) Urinary N-acetyl-beta-D-glucosaminidase and neopterin aid in the diagnosis of rejection and acute tubular necrosis in initially nonfunctioning kidney grafts. Nephron 84:228-235.
Liu Q, Nilsen-Hamilton M (1995) Identification of a new acute phase protein. J Biol Chem 270:22565-22570.
Molitoris BA (2003) Transitioning to therapy in ischemic acute renal failure. J Am Soc Nephrol 14:265-267.
Monier F, Surla A, Guillot M, Morel F (2000) Gelatinase isoforms in urine from bladder cancer patients. Clin Chim Acta 299:11-23.
Muramatsu Y, Tsujie M, Kohda Y, Pham B, Perantoni AO, Zhao H, Jo SK, Yuen PS, Craig L, Hu X, Star RA (2002) Early detection of cysteine rich protein 61 (CYR61, CCN1) in urine following renal ischemic reperfusion injury. Kidney Int 62:1601-1610. Penders J, Delanghe JR (2004) Alpha 1-microglobulin: clinical laboratory aspects and applications. Clin Chim Acta 346:107-118.
Stoesz SP, Gould MN (1995) Overexpression of neu-related lipocalin (NRL) in neu-initiated but not ras or chemically initiated rat mammary carcinomas. Oncogene 11:2233-2241.
Triebel S, Blaser J, Reinke H, Tschesche H (1992) A 25 kDa alpha 2-microglobulin-related protein is a component of the 125 kDa form of human gelatinase. FEBS Lett 314:386-388.
Tsuchida T, Eguchi N, Eguchi Y, Numabe A, Nakajima H, Oda H, Seiki K, Hakamada-Taguchi R, Urade Y, Uehara Y (2004) Lipocalin-type prostaglandin D synthase in urine in adriamycin-induced nephropathy of mice. Nephron Physiol 96:42-51.
Yan L, Borregaard N, Kjeldsen L, Moses MA (2001) The high molecular weight urinary matrix metalloproteinase (MMP) activity is a complex of gelatinase B/MMP-9 and neutrophil gelatinase-associated lipocalin (NGAL). Modulation of MMP-9 activity by NGAL. J Biol Chem 276:37258-37265.

### Items

1. A method of diagnosing, monitoring or determining the risk of developing acute renal failure in a human subject, wherein said method discriminates between a subject who does not have acute renal failure and is not at immediate risk of developing acute renal failure, and a subject who may have acute renal failure or is at risk of developing acute renal failure, said method comprising the steps of
   i) determining the concentration of human neutrophil gelatinase-associated lipocalin (NGAL) in a sample of bodily fluid from the subject, and
   ii) comparing said concentration with a predetermined cutoff value chosen so that an NGAL concentration below the cutoff value categorizes the subject as not having and not being at immediate risk of developing acute renal failure.
2. The method of item 1, wherein the sample is a urine sample and the cutoff value is a concentration of 250 ng/mL or a lower value, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL, or any value between 100 ng/mL and 50 ng/mL, such as 75 ng/mL.
3. The method of item 1, wherein the sample is a plasma or serum sample and the cutoff value is 250 ng/mL or a lower value, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL.
4. The monitoring method of any of the preceding items, comprising the further step of repeating steps i) and ii) of item 1 one or more times.
5. The monitoring method of any of the preceding items, comprising the further step of repeating steps i) and ii) of item 1 within 24 hours, e.g. within 12 hours, such as within 6 hours, e.g. within 3 hours, such as within 1 hour, e.g. within 30 minutes.
6. The monitoring method of any of the preceding items, comprising the further step of repeating steps i) and ii) of item1 after a treatment of acute renal failure has been initiated or completed.
7. The method of any of the preceding items, wherein the risk of developing acute renal failure is due to ischemic renal injury.
8. The method of any of the preceding items, wherein the risk of developing acute renal failure is due to a complication of an inflammatory, infective or neoplastic disease.
9. The method of any of the preceding items, wherein the risk of developing acute renal failure is due to critical illness of any cause requiring intensive care.
10. The method of any of the preceding items, wherein the risk of developing acute renal failure is due to a surgical intervention.
11. The method of any of the preceding items, wherein the risk of developing acute renal failure is due to the administration of a nephrotoxic agent.
12. The method of any of the preceding items, wherein NGAL is measured by means of a molecule that binds specifically to NGAL.
13. The method of any of the preceding items, wherein the bodily fluid is urine.
14. The method of any of the preceding items, wherein the bodily fluid is blood or plasma or serum.

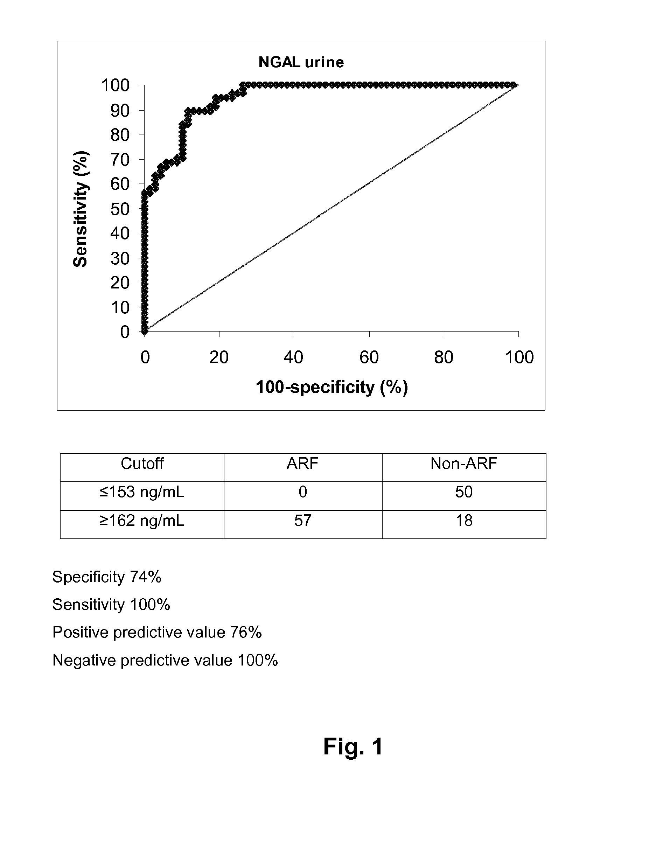

## Claims

1. A method of diagnosing, monitoring or determining the risk of developing acute renal failure in a human subject, wherein said method discriminates between a subject who does not have acute renal failure and is not at immediate risk of developing acute renal failure, and a subject who may have acute renal failure or is at risk of developing acute renal failure, said method comprising the steps of
i) determining the concentration of human neutrophil gelatinase-associated lipocalin (NGAL) in a sample of bodily fluid from the subject, and
ii) comparing said concentration with a predetermined cutoff value chosen so that an NGAL concentration below the cutoff value categorizes the subject as not having and not being at immediate risk of developing acute renal failure.

2. The method of claim 1, wherein the sample is a urine sample and the cutoff value is a concentration of 250 ng/mL or a lower value, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL, or any value between 100 ng/mL and 50 ng/mL, such as 75 ng/mL.

3. The method of claim 1, wherein the sample is a plasma or serum sample and the cutoff value is 250 ng/mL or a lower value, such any value between 250 ng/mL and 200 ng/mL, such as 225 ng/mL, or any value between 200 ng/mL and 150 ng/mL, such as 175 ng/mL or 160 ng/mL or 155 ng/mL, or any value between 150 ng/mL and 100 ng/mL, such as 125 ng/mL.

4. The monitoring method of any of the preceding claims, comprising the further step of repeating steps i) and ii) of claim 1 one or more times.

5. The monitoring method of any of the preceding claims, comprising the further step of repeating steps i) and ii) of claim 1 within 24 hours, e.g. within 12 hours, such as within 6 hours, e.g. within 3 hours, such as within 1 hour, e.g. within 30 minutes.

6. The monitoring method of any of the preceding claims, comprising the further step of repeating steps i) and ii) of claim 1 after a treatment of acute renal failure has been initiated or completed.

7. The method of any of the preceding claims, wherein the risk of developing acute renal failure is due to ischemic renal injury.

8. The method of any of the preceding claims, wherein the risk of developing acute renal failure is due to a complication of an inflammatory, infective or neoplastic disease.

9. The method of any of the preceding claims, wherein the risk of developing acute renal failure is due to critical illness of any cause requiring intensive care.

10. The method of any of the preceding claims, wherein the risk of developing acute renal failure is due to a surgical intervention.

11. The method of any of the preceding claims, wherein the risk of developing acute renal failure is due to the administration of a nephrotoxic agent.

12. The method of any of the preceding claims, wherein NGAL is measured by means of a molecule that binds specifically to NGAL.

13. The method of any of the preceding claims, wherein the bodily fluid is urine.

14. The method of any of the preceding claims, wherein the bodily fluid is blood or plasma or serum.
